# EUROPEAN PATENT SPECIFICATION

(11) **EP 3 045 444 B1**
(45) Date of publication and mention of the grant of the patent: **12.05.2021**
(21) Application number: 15750614.8
(22) Date of filing: 29.07.2015
(51) Int. Cl.: C07C 47/58, C07C 45/36

(54) **METHOD OF PREPARING VANILLIN**
VERFAHREN ZUR HERSTELLUNG VON VANILLIN
PROCÉDÉ DE PRÉPARATION DE VANILLINE

(30) Priority: 30.06.2014 CN 201410304472
(43) Date of publication of application: 20.07.2016
(73) Proprietor: JIAXING ZHONGHUA CHEMICAL CO., LTD., Nanhu District Jiaxing City, Zhejiang 314000 (CN)
(72) Inventor: MAO, Haifang, Jiaxing Zhejiang 314000 (CN); WANG, Lizhi, Jiaxing Zhejiang 314000 (CN); LIU, Zhenjiang, Jiaxing Zhejiang 314000 (CN); YAO, Yueliang, Jiaxing Zhejiang 314000 (CN); WANG, Chaoyang, Jiaxing Zhejiang 314000 (CN); WU, Jianxin, Jiaxing Zhejiang 314000 (CN); ZHAO, Feifei, Jiaxing Zhejiang 314000 (CN)
(74) Representative: Mewburn Ellis LLP
(86) International application number: PCT/CN2015/085408
(87) International publication number: WO 2016/000664

(56) References cited:
- WO-A1-2010/003161
- WO-A1-2010/003161
- CN-A- 1 167 750
- CN-A- 1 289 836
- CN-A- 102 206 146
- CN-A- 102 206 146
- CN-A- 103 626 643
- CN-B- 102 091 637
- CN-B- 102 701 927
- GB-A- 645 430
- JP-A- S63 104 937
- US-A- 3 544 621
- US-A- 5 861 286
- LUCIANA?A. PARREIRA ET AL: "Palladium-Catalyzed Aerobic Oxidation of Naturally Occurring Allylbenzenes as a Route to Valuable Fragrance and Pharmaceutical Compounds", ADVANCED SYNTHESIS & CATALYSIS, vol. 352, no. 9, 1 June 2010 (2010-06-01), pages 1533-1538, XP055469585, DE ISSN: 1615-4150, DOI: 10.1002/adsc.201000050

## Description

### FIELD OF THE INVENTION

The present invention pertains to chemical industry field, and especially relates to an eugenol, in particular, a preparation method of vanillin.

### BACKGROUND OF THE INVENTION

Vanillin, chemical name 4-hydroxy-3-methoxybenzaldehyde, is a broad-spectrum spice with rich milk flavor. It is mostly used in the industries of food, daily necessities, medical treatment, wine making, tobacco and the like. Vanillin is obtainable by plant extraction, biotransformation and chemical synthesis methods.

Plant extraction method is the most direct way to get natural vanillin. Vanillin has been found in many essential oils and plants, and its content in Vanilla beans is the highest. Vanillin obtained by the method belongs to natural products, and has heavy aroma. However, artificial pollination of flowers is needed in vanilla bean planting process, which requires high labor intensity, so vanilla beans cannot be planted in large scale. Therefore, this method can not meet market demand.

Biotransformation has the advantages of being clean, less pollution, safe production, almost natural product. However, the reaction system is too complicated, product vanillin has certain toxicity to enzyme or microorganism, yields and conversion rates are low, separation process of vanillin is complex, cost is high, and residue of metabolites generated in biotransformation also reduces quality and safety of products.

Vanillin on the market is mostly obtained by the chemical synthesis methods, including safrole method, lignin method, p-cresol method, 4-methylguaiacol method, p-hydroxybenzaldehyde method, guaiacol method, etc. Currently, the chemical synthesis method commonly used is glyoxylic acid method, which comprises condensing guaiacol and glyoxylic acid under basic conditions to form 3-methoxy-4-hydroxymandelic acid, oxidizing in the presence of a catalyst to form 3-methoxy-4-hydroxybenzoylformic acid, decarboxylating the product to form vanillin, and then carrying out extraction, distillation, crystallization with dilute ethanol to give pure vanillin. However, the method is prone to production of large amounts of wastewater containing phenol when applied in industrial production, thereby causing environmental pollution. The rest methods of preparing vanillin are all involved at home and abroad, but the above methods either have a long process route and high requirements for equipment, and cause severe environmental pollution, or are low in reaction efficiency, complex in separation of products, and poor in quality of aroma.

Natural-grade vanillin prepared with eugenol as raw material has enticing aroma, food safety, and high practical value, and thus attracts wide attention and in-depth research. Chinese patent CN1289836A published on April 4, 2001 reported a method of preparing vanillin from clove oil, developed by Shanghai Tianxiang Fine Chemical Co., Ltd., comprising adding sodium hydroxide in clove oil, then adding an oxidizing agent and carbonyl-based mixed catalyst, performing oxidation at 100-300°C, and finally obtaining vanillin with a yield of over 50%, wherein the oxidizing agent used is potassium permanganate, p-aminobenzenesulfonic acid and nitrobenzene, and all reaction temperatures are above 100°C. The oxidizing agents used in the invention such as potassium permanganate are all oxidizing agents in general use traditionally. The waste generated by the reaction is difficult to deal with, resulting in serious environment pollution. The method is being phased out gradually.

In methods of synthesizing vanillin with eugenol as a raw material, traditionally eugenol is isomerized first and then oxidized. Namely, eugenol is first isomerized into isoeugenol under the action of sodium hydroxide or potassium hydroxide, and then oxidized into vanillin under the action of oxidants. For example, Chinese patent No. CN102206146A published on October 5, 2011 and Chinese patent No. CN103641698A published on March 19, 2014 both disclose methods of isomerizing eugenol into isoeugenol and then preparing natural vanillin.

CN102701927B describes a method for producing vanillin. The method comprises the steps of: (1) mixing clove leaf oil, water and potassium hydroxide, and heating for performing an isomerization reaction; (2) cooling an isomerization reaction product, adding water and an oxidant into the product, and heating for performing an oxidation reaction; (3) adjusting the pH value of an oxidization reaction product to acid, cooling, leaching, and distilling under reduced pressure to obtain crude vanillin; and (4) performing alcohol crystallization on the crude vanillin to obtain finished vanillin.

US 3,544,621 A describes a process of preparing vanillin comprising isomerizing eugenol in the presence of an alkali metal hydroxide in aqueous solution, oxidizing the isomer in the solution by means of sodium meta-nitrobenzene sulfonate, acidifying the oxidate with sulfuric acid and extracting vanillin from the acidic mixture by means of a water-immiscible organic solvent, further including: (a) recovering the aqueous phase from the extraction step and concentrating said phase to obtain a concentrated solution containing 4-6 parts by weight of water per part by weight of the initial eugenol; (b) intimately admixing the concentrated solution with 2.5-3 parts by weight of concentrated sulfuric acid per 1 weight part initial eugenol; (c) heating the acidic solution to boiling; (d) adding to the solution concentrated nitric acid in an amount of at least 4 mols per 3 mols sodium metanitrobenzene sulfonate used for oxidizing the isomer and continuing boiling until the initial amount of sulfonate is substantially restored. (e) neutralizing the solution obtained from step (d) with aqueous alkali metal hydroxide and cycling the neutralized solution for use in the oxidation of further amounts of isoeugenol

CN 1167750 discloses a process of production of vanillin by oxidation of the methyl of 4-methyl-2-methoxyphenol with oxygen , in presence of mixed salts of cobalt salt and copper salt.

WO 2010/003161 A1 describes a process for the oxidative cleavage of the ethylenic double bonds of vinylaromatics.

### DESCRIPTION OF THE INVENTION

In view of the above defects existing in the prior art, the technical problem to be solved by the present invention is to provide a method of preparing vanillin. The method is to solve the technical problems of complex process, high cost, and great pollution of synthesis of vanillin from eugenol in the prior art.

The invention provides a method of preparing vanillin, comprising
1) reacting eugenol with oxygen gas in the presence of a strong alkali and a catalyst to obtain vanillin salt; and
2) reacting the vanillin salt with an acid to obtain vanillin;
wherein the catalyst is cobalt salt, mixed salts of cobalt salt and copper salt, or mixed salts of cobalt salt and nickel salt; and wherein the strong alkali is sodium hydroxide or potassium hydroxide, wherein the method comprises weighing eugenol, a strong alkali, a catalyst, and a first organic solvent, respectively, the molar ratio of the strong alkali to eugenol being 1 : 2-15, the amount of the catalyst being 0.1% to 2.0% of the weight of eugenol, and the volume of the first organic solvent being 3 to 10 folds of the volume of eugenol; adding the above substances to a reactor, respectively, the reaction pressure being 0.01-0.30 MPa, heating to 60-120°C under stirring, then beginning to introduce oxygen gas to control partial pressure of oxygen gas within 0.01-1 MPa, and performing oxidation reaction for 4-30 h; cooling to room temperature, adding water with the volume ratio of water to the first solvent being 0.5 to 2, adjusting to neutrality with an acid solution, recovering the first organic solvent, extracting the water phase with a second organic solvent, carrying out reduced pressure distillation, crystallizing with a mixed solvent of ethanol and water, and drying to give vanillin.

Further, the second organic solvent is toluene or ethylbenzene.

Further, the first organic solvent is methanol or ethanol.

Further, the volume ratio of ethanol to water in the mixed solvent of ethanol and water is 0.3-5 : 1.

Further, the weight ratio of cobalt salt to copper salt in the mixed salts of cobalt salt and copper salt is an arbitrary ratio.

Further, the weight ratio of cobalt salt to nickel salt in the mixed salts of cobalt salt and nickel salt is an arbitrary ratio.

Further, the acid solution is hydrochloric acid or sulfuric acid solution whose molar concentration is 1 to 3 mol/L.

The reaction equation of the present invention is as follows:

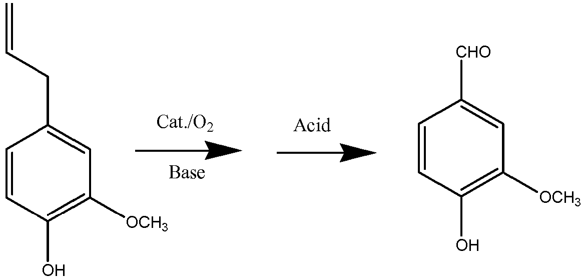

The present invention provides a method of preparing vanillin from eugenol by oxidation reaction with introduced oxygen gas at a reaction pressure of 0.01 to 0.30 MPa, a reaction temperature of 60 to 120°C, and a partial pressure of oxygen gas of 0.01 to 0.1 MPa, in the presence of a strong alkali and a catalyst being cobalt salt, mixed salts of cobalt salt and copper salt, or mixed salts of cobalt salt and nickel salt.

In comparison to existing technology, the present invention represents notable technical progress. In the present invention, vanillin is catalytically synthesized from eugenol directly in one step, without protection of phenolic hydroxyl group of eugenol, isomerization of eugenol or other processes. The invention is simple in process, convenient for post treatment, high in yield, and suitable for industrialized production, and the aroma of the product is good. The present invention additionally has the features of simple operation, low cost and less pollution.

### EMBODIMENTS

The invention provides a method of preparing vanillin, comprising
1) reacting eugenol with oxygen gas in the presence of a strong alkali and a catalyst to obtain vanillin salt; and
2) reacting the vanillin salt with an acid to obtain vanillin;
wherein the catalyst is cobalt salt, mixed salts of cobalt salt and copper salt, or mixed salts of cobalt salt and nickel salt; and wherein the strong alkali is sodium hydroxide or potassium hydroxide, wherein the method comprises:weighing eugenol, a strong alkali, a catalyst, and a first organic solvent, respectively, the molar ratio of the strong alkali to eugenol being 1 : 2-15, the amount of the catalyst being 0.1 % to 2.0% of the weight of eugenol, and the volume of the first organic solvent being 3 to 10 folds of the volume of eugenol;adding the above substances to a reactor, respectively, the reaction pressure being 0.01-0.30 MPa, heating to 60-120°C under stirring, then beginning to introduce oxygen gas to control partial pressure of oxygen gas within 0.01-1 MPa, and performing oxidation reaction for 4-30 h;cooling to room temperature, adding water with the volume ratio of water to the first solvent being 0.5 to 2, adjusting to neutrality with an acid solution, recovering the first organic solvent, extracting the water phase with a second organic solvent, carrying out reduced pressure distillation, crystallizing with a mixed solvent of ethanol and water, and drying to give vanillin.

According to the present invention, eugenol is reacted with oxygen gas in the presence of a strong alkali and a catalyst to obtain vanillin salt, wherein the strong alkali is sodium hydroxide or potassium hydroxide; the reaction solvent is preferably methanol or ethanol, more preferably methanol; the catalyst is cobalt salt, mixed salts of cobalt salt and copper salt, or mixed salts of cobalt salt and nickel salt. For the mixed salts of cobalt salt and copper salt, there is no special requirement for the ratio of both in the mixed salts in the present invention, and it may be any ratio. Preferably, the mass ratio of cobalt salt to copper salt is 1 : (0.1-1). For the mixed salts of cobalt salt and nickel salt, there is no special requirement for the ratio of both in the mixed salts in the present application. Preferably, the mass ratio of cobalt salt to nickel salt is 1 : (0.1-1).

Molar ratio of eugenol to the strong alkali is 1 : (2-15), preferably 1 : (3-10). Mass ratio of eugenol to the catalyst is preferably 100 : (0.1-3), more preferably, 100 : (0.2-2). Amount ratio of eugenol to the solvent is 1 g : (3-10) mL, more preferably, 1 g : (4-8) mL. The partial pressure of oxygen gas in the reaction system is preferably 0.01 to 1 MPa, more preferably 0.05 to 0.5 MPa, most preferably, 0.1 to 0.3 MPa. The reaction temperature is preferably 60 to 120°C, more preferably 80 to 110°C, most preferably 85 to 110°C.

In the present invention, the obtained vanillin salt is further reacted with an acid to give vanillin. There is no special requirement for the acid. All acids well known in the art may be used, preferably hydrochloric acid or sulfuric acid. Molar concentration of the acid is preferably 1 to 3 mol/L.

In the preparation method of the present invention, by introducing a special catalyst to the process of preparing vanillin from eugenol, isomerization and oxidation of eugenol can be completed in one step. Moreover, the process is simple, post treatment is convenient and yield is high.

Examples of the present invention are merely provided to illustrate the particular method.

### Example 1

To a 1000 ml autoclave, 73.8 g of eugenol, 90 g of sodium hydroxide, 0.075 g of cobalt acetate, and 0.075 g nickel acetate were added, and then 450 ml methanol was added. The autoclave was kept with good sealability, heated to 60 to 65°C under stirring, and then introduced with oxygen gas. Oxidation reaction was carried out with partial pressure of oxygen gas controlled at 0.03 MPa. After the reaction was finished, the product was cooled to room temperature, added with 300 ml water, and neutralized to neutrality with hydrochloric acid. Methanol was recovered, and then the water phase was extracted with toluene. The extractive solution was subjected to toluene recovering, reduced pressure distillation, and crystallization with a mixed solvent of ethanol and water. The resulting product was dried to obtain 36.0 g of vanillin in form of white needle-like crystal, with a yield of 52.68%, and a purity of 99.8%.

### Example 2

To a 1000 ml autoclave, 73.8 g of eugenol, 30 g of sodium hydroxide, 0.22 g of cobalt acetate, and 0.22 g copper acetate were added, and then 300 ml methanol was added. The autoclave was kept with good sealability, heated to 80 to 85°C under stirring, and then introduced with oxygen gas. Oxidation reaction was carried out with partial pressure of oxygen gas controlled at 0.05 MPa. After the reaction was finished, the product was cooled to room temperature, added with 300 ml water, and neutralized to neutrality with hydrochloric acid. Methanol was recovered, and then the water phase was extracted with toluene. The extractive solution was subjected to toluene recovering, reduced pressure distillation, and crystallization with a mixed solvent of ethanol and water. The resulting product was dried to obtain 33.9 g of vanillin in form of white needle-like crystal, with a yield of 49.9%, and a purity of 99.9%.

### Example 3

To a 1000 ml autoclave, 73.8 g of eugenol, 180 g of sodium hydroxide, and 1.48 g of cobalt acetate were added, and then 540 ml methanol was added. The autoclave was kept with good sealability, heated to 100 to 105°C under stirring, and then introduced with oxygen gas. Oxidation reaction was carried out with partial pressure of oxygen gas controlled at 0.01 MPa. After the reaction was finished, the product was cooled to room temperature, added with 450 ml water, and neutralized to neutrality with hydrochloric acid. Methanol was recovered, and then the water phase was extracted with toluene. The extractive solution was subjected to toluene recovering, reduced pressure distillation, and crystallization with a mixed solvent of ethanol and water. The resulting product was dried to obtain 41.4 g of vanillin in form of white needle-like crystal, with a yield of 60.5%, and a purity of 99.9%.

### Example 4

To a 1000 ml autoclave, 73.8 g of eugenol, 90 g of potassium hydroxide, 0.22 g of cobalt acetate, and 0.07 g nickel acetate were added, and then 375 ml methanol was added. The autoclave was kept with good sealability, heated to 115 to 120°C under stirring, and then introduced with oxygen gas. Oxidation reaction was carried out with partial pressure of oxygen gas controlled at 0.1 MPa. After the reaction was finished, the product was cooled to room temperature, added with 360 ml water, and neutralized to neutrality with hydrochloric acid. Methanol was recovered, and then the water phase was extracted with toluene. The extractive solution was subjected to toluene recovering, reduced pressure distillation, and crystallization with a mixed solvent of ethanol and water. The resulting product was dried to obtain 33.1 g of vanillin in form of white needle-like crystal, with a yield of 48.2%, and a purity of 99.7%.

The above illustration by examples is merely to help understanding of the method of the present invention and its core concept.

## Claims

1. A method of preparing vanillin, comprising
1) reacting eugenol with oxygen gas in the presence of a strong alkali and a catalyst to obtain vanillin salt; and
2) reacting vanillin salt with an acid to obtain vanillin;
wherein the catalyst is cobalt salt, mixed salts of cobalt salt and copper salt, or mixed salts of cobalt salt and nickel salt; and
wherein the strong alkali is sodium hydroxide or potassium hydroxide,
wherein the method comprises:weighing eugenol, a strong alkali, a catalyst, and a first organic solvent, respectively, the molar ratio of the strong alkali to eugenol being 1 : 2-15, the amount of the catalyst being 0.1% to 2.0% of the weight of eugenol, and the volume of the first organic solvent being 3 to 10 folds of the volume of eugenol;
adding the above substances to a reactor, respectively, the reaction pressure being 0.01-0.30 MPa, heating to 60-120°C under stirring, then beginning to introduce oxygen gas to control partial pressure of oxygen gas within 0.01-1 MPa, and performing oxidation reaction for 4-30 h;
cooling to room temperature, adding water with the volume ratio of water to the first solvent being 0.5 to 2, adjusting to neutrality with an acid solution, recovering the first organic solvent, extracting the water phase with a second organic solvent, carrying out reduced pressure distillation, crystallizing with a mixed solvent of ethanol and water, and drying to give vanillin.

2. The method of preparing vanillin according to claim 1, **characterized in that** the second organic solvent is toluene or ethylbenzene.

3. The method of preparing vanillin according to claim 1, **characterized in that** the first organic solvent is methanol or ethanol.

4. The method of preparing vanillin according to claim 1, **characterized in that** the volume ratio of ethanol to water in the mixed solvent of ethanol and water is 0.3-5 : 1.

5. The method of preparing vanillin according to claim 1, **characterized in that** the weight ratio of cobalt salt to copper salt in the mixed salts of cobalt salt and copper salt is an arbitrary ratio.

6. The method of preparing vanillin according to claim 1, **characterized in that** the weight ratio of cobalt salt to nickel salt in the mixed salts of cobalt salt and nickel salt is an arbitrary ratio.

7. The method of preparing vanillin according to claim 1, **characterized in that** the acid solution is hydrochloric acid or sulfuric acid solution whose molar concentration is 1 to 3 mol/L.

## Patentansprüche

1. Verfahren zur Herstellung von Vanillin, wobei das Verfahren Folgendes umfasst:
1) das Umsetzen von Eugenol mit Sauerstoffgas in Gegenwart einer starken Base und eines Katalysators zum Erhalt von Vanillinsalz; und
2) das Umsetzen von Vanillinsalz mit einer Säure zum Erhalt von Vanillin;
wobei der Katalysator Cobaltsalz, ein Salzgemisch aus Cobaltsalz und Kupfersalz oder ein Salzgemisch aus Cobaltsalz und Nickelsalz ist; und
wobei die starke Base Natriumhydroxid oder Kaliumhydroxid ist,
wobei das Verfahren Folgendes umfasst: das Einwiegen von Eugenol, einer starken Base, eines Katalysators und eines ersten organischen Lösungsmittels, wobei das Molverhältnis der starken Base zu Eugenol 1 : 2-15 beträgt, die Menge des Katalysators 0,1 bis 2,0 Gew.-% des Eugenols entspricht und das Volumen des ersten organischen Lösungsmittels dem 3- bis 10-Fachen des Volumens des Eugenols entspricht;
das Zusetzen der oben angeführten Substanzen zu einem Reaktor, wobei der Reaktionsdruck 0,01 bis 0,30 MPa beträgt, das Erhitzen auf 60 bis 120 °C unter Rühren, anschließend das Beginnen des Einbringens von Sauerstoffgas zur Steuerung des Partialdrucks des Sauerstoffgases innerhalb eines Bereichs von 0,01 bis 1 MPa und das 4- bis 30-stündige Durchführen einer Oxidationsreaktion;
das Abkühlen auf Raumtemperatur, das Zusetzen von Wasser, wobei das Volumenverhältnis von Wasser zu dem ersten Lösungsmittel 0,5 bis 2 beträgt, das Einstellen auf Neutralität mit einer sauren Lösung, das Gewinnen des ersten organischen Lösungsmittels, das Extrahieren der wässrigen Phase mit einem zweiten organischen Lösungsmittel, das Durchführen von Unterdruckdestillation, das Kristallisieren mit einem Lösungsmittelgemisch aus Ethanol und Wasser und das Trocknen zum Erhalt von Vanillin.

2. Verfahren zur Herstellung von Vanillin nach Anspruch 1, **dadurch gekennzeichnet, dass** das zweite organische Lösungsmittel Toluol oder Ethylbenzol ist.

3. Verfahren zur Herstellung von Vanillin nach Anspruch 1, **dadurch gekennzeichnet, dass** das erste organische Lösungsmittel Methanol oder Ethanol ist.

4. Verfahren zur Herstellung von Vanillin nach Anspruch 1, **dadurch gekennzeichnet, dass** das Volumenverhältnis von Ethanol zu Wasser in dem Lösungsmittelgemisch aus Ethanol und Wasser 0,3-5 : 1 beträgt.

5. Verfahren zur Herstellung von Vanillin nach Anspruch 1, **dadurch gekennzeichnet, dass** das Gewichtsverhältnis von Cobaltsalz zu Kupfersalz in dem Salzgemisch aus Cobaltsalz und Kupfersalz ein beliebiges Verhältnis ist.

6. Verfahren zur Herstellung von Vanillin nach Anspruch 1, **dadurch gekennzeichnet, dass** das Gewichtsverhältnis von Cobaltsalz zu Nickelsalz in dem Salzgemisch aus Cobaltsalz und Nickelsalz ein beliebiges Verhältnis ist.

7. Verfahren zur Herstellung von Vanillin nach Anspruch 1, **dadurch gekennzeichnet, dass** die saure Lösung Salzsäure- oder Schwefelsäurelösung ist, deren Molkonzentration 1 bis 3 mol/l beträgt.

## Revendications

1. Procédé de préparation de vanilline, comprenant
1) la réaction d'eugénol avec de l'oxygène gazeux en présence d'un alcali fort et d'un catalyseur pour obtenir du sel de vanilline ; et
2) la réaction de sel de vanilline avec un acide pour obtenir de la vanilline ;
dans lequel le catalyseur est un sel de cobalt, des sels mélangés de sel de cobalt et de sel de cuivre, ou des sels mélangés de sel de cobalt et de sel de nickel ; et
dans lequel l'alcali fort est de l'hydroxyde de sodium ou hydroxyde de potassium,
dans lequel le procédé comprenant les étapes consistant à : peser de l'eugénol, un alcali fort, un catalyseur, et un premier solvant organique, respectivement, le rapport molaire entre l'alcali fort et l'eugénol étant de 1:2-15, la quantité du catalyseur étant de 0,1 % à 2,0 % du poids d'eugénol, et le volume du premier solvant organique étant de 3 à 10 fois le volume d'eugénol ;
ajouter les substances ci-dessus dans un réacteur, respectivement, la pression de réaction étant de 0,01-0,30 MPa, chauffer à 60-120°C tout en remuant, puis commencer à introduire de l'oxygène gazeux pour commander une pression partielle d'oxygène gazeux dans la plage de 0,01-1 MPa, et effectuer une réaction d'oxydation pendant 4-30 h ;
refroidir à température ambiante, ajouter de l'eau avec le rapport volumique entre l'eau et le premier solvant qui est de 0,5 à 2, ajuster à neutralité à l'aide d'une solution acide, récupérer le premier solvant organique, extraire la phase aqueuse à l'aide d'un second solvant organique, réaliser une distillation sous pression réduite, cristalliser à l'aide d'un solvant mélangé d'éthanol et d'eau, et sécher pour donner de la vanilline.

2. Procédé de préparation de vanilline selon la revendication 1, **caractérisé en ce que** le second solvant organique est du toluène ou de l'éthylbenzène.

3. Procédé de préparation de vanilline selon la revendication 1, **caractérisé en ce que** le premier solvant organique est du méthanol ou de l'éthanol.

4. Procédé de préparation de vanilline selon la revendication 1, **caractérisé en ce que** le rapport volumique entre l'éthanol et l'eau dans le solvant mélangé d'éthanol et d'eau est de 0,3-5:1.

5. Procédé de préparation de vanilline selon la revendication 1, **caractérisé en ce que** le rapport en poids entre le sel de cobalt et le sel de cuivre dans les sels mélangés de sel de cobalt et de sel de cuivre est un ratio arbitraire.

6. Procédé de préparation de vanilline selon la revendication 1, **caractérisé en ce que** le rapport en poids entre le sel de cobalt et le sel de nickel dans les sels mélangés de sel de cobalt et de sel de nickel est un rapport arbitraire.

7. Procédé de préparation de vanilline selon la revendication 1, **caractérisé en ce que** la solution acide est une solution d'acide chlorhydrique ou d'acide sulfurique dont une concentration molaire est de 1 à 3 mol/L.
